(19) Europäisches Patentamt
European Patent Office
Office européen des brevets

(11) **EP 4 609 785 A1**

(12) **EUROPEAN PATENT APPLICATION**
published in accordance with Art. 153(4) EPC

(43) Date of publication:
**03.09.2025 Bulletin 2025/36**

(21) Application number: **23881746.4**

(22) Date of filing: **20.10.2023**

(51) International Patent Classification (IPC):
***A61B 5/022*** (2006.01)   ***A61B 5/021*** (2006.01)

(52) Cooperative Patent Classification (CPC):
**A61B 5/00; A61B 5/021; A61B 5/022**

(86) International application number:
**PCT/CN2023/125632**

(87) International publication number:
**WO 2024/088168 (02.05.2024 Gazette 2024/18)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **25.10.2022 CN 202211312253**

(71) Applicant: **Huawei Technologies Co., Ltd.
Shenzhen, Guangdong 518129 (CN)**

(72) Inventors:
• **CHEN, Qing
Shenzhen, Guangdong 518129 (CN)**
• **DING, Li
Shenzhen, Guangdong 518129 (CN)**
• **LIU, Chuangchuang
Shenzhen, Guangdong 518129 (CN)**
• **CHEN, Baoyang
Shenzhen, Guangdong 518129 (CN)**
• **FENG, Rongkai
Shenzhen, Guangdong 518129 (CN)**

(74) Representative: **Gill Jennings & Every LLP
The Broadgate Tower
20 Primrose Street
London EC2A 2ES (GB)**

(54) **SMART WATCH AND BLOOD PRESSURE MEASUREMENT METHOD**

(57)    A smartwatch and a blood pressure measurement method are provided, so that a plurality of pulse waves of a radial artery of a user can be accurately obtained, and blood pressure of the user can be determined based on the plurality of pulse waves. Therefore, accuracy of blood pressure measurement can be improved, and user experience is further improved. The smartwatch includes a watch body, an airbag, and a sensor. The watch body is electrically connected to the sensor. The sensor is disposed on an inner wall of the airbag. When the smartwatch is worn on a wrist of the user, the airbag and the sensor cover the radial artery of the wrist. The sensor is configured to measure the plurality of pulse waves of the radial artery when the airbag is under different pressure strength. The watch body is configured to determine the blood pressure of the user based on the plurality of pulse waves.

FIG. 2

**EP 4 609 785 A1**

## Description

This application claims priority to Chinese Patent Application No. 202211312253.3, filed with the China National Intellectual Property Administration on October 25, 2022 and entitled "SMARTWATCH AND BLOOD PRESSURE MEASUREMENT METHOD", which is incorporated herein by reference in its entirety.

## TECHNICAL FIELD

Embodiments of this application relate to the field of electronic devices, and in particular, to a smartwatch and a blood pressure measurement method.

## BACKGROUND

As a chronic disease with a largest number of patients in the world, hypertension is particularly harmful. To meet a requirement of a user for measuring blood pressure of the user anytime and anywhere, portable electronic devices like a smartwatch, a smart vest, and a smart band that have a blood pressure measurement function emerge.

Currently, an airbag may be integrated into the smartwatch, to improve convenience of blood pressure measurement. Specifically, the smartwatch usually includes a watch body, the airbag, and a sensor. In addition, one end of the sensor is connected to one end of the airbag, and the other end of the sensor is connected to the watch body. When the watch body inflates the airbag, the airbag squeezes a wrist of the user. In this case, the sensor may collect a signal from the airbag, and send the collected signal to the watch body, so that the watch body analyzes the signal, to obtain the blood pressure of the user.

However, the wrist usually includes a radial artery and an ulnar artery. Wrist sizes of some users are large, and the airbag may cover only the radial artery. In this case, signals collected by the sensor are all signals related to the radial artery. Wrist sizes of some other users are small, and the airbag may cover the radial artery and the ulnar artery. In this case, signals collected by the sensor include both a signal related to the radial artery and a signal related to the ulnar artery, and signal types are inconsistent. As a result, finally obtained blood pressure of the user is inaccurate, and user experience is reduced.

## SUMMARY

Embodiments of this application provide a smartwatch and a blood pressure measurement method, so that a plurality of pulse waves of a radial artery of a user can be accurately obtained, and blood pressure of the user can be determined based on the plurality of pulse waves. Therefore, accuracy of blood pressure measurement can be improved, and user experience is further improved.

A first aspect of embodiments of this application provides a smartwatch. The smartwatch includes a watch body and two watchbands. One end of one watchband is connected to one end of the watch body, and one end of the other watchband is connected to the other end of the watch body. At least a processor and an air pump are disposed inside the watch body. An airbag and a sensor are disposed inside one watchband. In a cavity of the watchband, one end of the airbag is connected to one end of the watch body through a part of the watchband. Because a cavity is also disposed in the airbag, the sensor may be disposed in the cavity of the airbag. The sensor is attached to an inner wall of the airbag. The sensor is electrically connected to the processor. When the smartwatch is worn by a user on a wrist of the user, the airbag and the sensor may cover a radial artery of the wrist of the user.

After receiving a blood pressure measurement instruction delivered by the user, the processor in the watch body may control, based on the instruction, the air pump in the watch body to adjust pressure strength inside the airbag, so that the airbag squeezes the radial artery of the user. In a process in which the pressure strength of the airbag continuously changes, the sensor may detect the radial artery of the user, to obtain a plurality of pulse waves of the radial artery through measurement.

After obtaining the plurality of pulse waves of the radial artery that are sent by the sensor, the processor in the watch body may process the plurality of pulse waves, to obtain blood pressure of the user, for example, systolic pressure and diastolic pressure of the user.

The smartwatch includes the watch body, the airbag, and the sensor. The watch body is electrically connected to the sensor. The sensor is disposed on the inner wall of the airbag. When the smartwatch is worn on the wrist of the user, the airbag and the sensor cover the radial artery of the wrist. Specifically, the watch body may first increase pressure strength of the airbag, so that the airbag squeezes the radial artery of the user. Therefore, the sensor may measure a plurality of pulse waves of the radial artery when the airbag is under different pressure strength. Then, the watch body may determine the blood pressure of the user based on the plurality of pulse waves of the radial artery. After the smartwatch is worn on the wrist of the user, the sensor in the airbag may directly cover the radial artery of the user, but does not cover an ulnar artery of the user. When the watch body inflates the airbag to make the airbag squeeze the radial artery of the user, the sensor may collect the plurality of pulse waves that are related only to the radial artery. Therefore, the blood pressure of the user determined by the watch body based on the plurality of pulse waves of the radial artery is accurate enough, and user experience can be improved.

In a possible implementation, a distance between a top of a target area of the airbag and a top of the airbag is greater than a first threshold. A length of the target area is greater than a second threshold. A width of the target area is less than a product of a width of the airbag and a preset coefficient. The target area is an area occupied by the sensor. In the foregoing implementation, an area may be delimited on an inner surface of the airbag to place the sensor. The area is referred to as the target area. A size of the target area may be set in the following manner: In the airbag, the distance between the top of the target area and the top of the airbag is greater than the first threshold, the length of the target area is greater than the second threshold, and the width of the target area is less than the product of the width of the airbag and the preset coefficient. In this way, for a user with a large wrist size, a user with a normal wrist size, and a user with a small wrist size, the sensor may cover radial arteries in wrists of all users as many as possible, and the pulse wave of the radial artery collected by the sensor may be an effective signal.

In a possible implementation, the watch body is further configured to increase the pressure strength of the airbag, so that the airbag squeezes the radial artery until the plurality of pulse waves of the radial artery from the sensor meet a preset condition. The plurality of pulse waves are in one-to-one correspondence with a plurality of pieces of pressure strength of the airbag. In the foregoing implementation, after receiving the blood pressure measurement instruction delivered by the user, the processor in the watch body may control, based on the instruction, the air pump in the watch body to continuously increase the pressure strength inside the airbag (that is, inflate the airbag), to obtain the plurality of pieces of pressure strength of the airbag. The airbag under the plurality of pieces of pressure strength may continuously squeeze a radial artery of a wrist of the user. In this process, the sensor may detect the radial artery, to obtain the plurality of pulse waves of the radial artery, and then send the plurality of pulse waves to the processor. The plurality of pulse waves of the radial artery are in one-to-one correspondence with the plurality of pieces of pressure strength of the airbag. After determining that the plurality of pulse waves of the radial artery meet the preset condition, the processor no longer increases the pressure strength of the airbag (that is, no longer inflates the airbag).

In a possible implementation, the watch body is configured to: continuously increase the pressure strength of the airbag until an amplitude of a latest pulse wave in the plurality of pulse waves is less than a preset percentage of an amplitude of a target pulse wave, where the amplitude of the target pulse wave is the largest in amplitudes of the plurality of pulse waves; or intermittently increase the pressure strength of the airbag until an amplitude of a latest pulse wave in the plurality of pulse waves is less than an amplitude of a previous pulse wave; or continuously increase the pressure strength of the airbag until an amplitude of a latest pulse wave in the plurality of pulse waves is within a preset range. In the foregoing implementation, the processor may determine, in a plurality of manners, whether the plurality of pulse waves meet the preset condition: (1) The processor may continuously increase the pressure strength of the airbag, that is, continuously inflate the airbag. If the amplitude of the latest pulse wave from the sensor is less than the preset percentage of the amplitude of the target pulse wave, it indicates that the radial artery of the user is in a closed state, and the processor deflates the airbag. It should be noted that the target pulse wave is a pulse wave with a largest amplitude in the plurality of pulse waves collected by the sensor, and the target pulse wave is usually a pulse wave previous to the latest pulse wave. (2) The processor may intermittently increase the pressure strength of the airbag, that is, first inflate the airbag, then neither inflate nor deflate the airbag, then continue to inflate the airbag, and then neither inflate nor deflate the airbag. If the amplitude of the latest pulse wave is less than the amplitude of the previous pulse wave, it indicates that the radial artery of the user is in a flat state, and the processor deflates the airbag. (3) The processor may continuously increase the pressure strength of the airbag, that is, continuously inflate the airbag. If the amplitude of the latest pulse wave from the sensor is within the preset range, it indicates that the radial artery of the user is in a slightly pressed state, and the processor neither inflates nor deflates the airbag.

In a possible implementation, the watch body is configured to: determine a transfer function of the radial artery for a pulse wave and a transfer function of skin of the user for a pulse wave based on the amplitudes of the plurality of pulse waves; and calculate the plurality of pulse waves based on the transfer functions, to obtain the blood pressure of the user. In the foregoing implementation, the processor may construct a feature vector (where the feature vector may indicate a waveform change of the pulse wave of the radial artery, the feature vector includes a plurality of elements, and the plurality of elements are the amplitudes of the plurality of pulse waves of the radial artery) of the pulse wave of the radial artery based on the amplitudes of the plurality of pulse waves of the radial artery. Then, the processor may determine, based on the feature vector of the pulse wave of the radial artery, the transfer function of the radial artery of the user for the pulse wave and the transfer function of the skin of the user for the pulse wave. After obtaining the transfer function of the radial artery for the pulse wave and the transfer function of the skin for the pulse wave, the processor may calculate the plurality of pulse waves based on the transfer function of the radial artery for the pulse wave and the transfer function of the skin for the pulse wave, to obtain the blood pressure of the user. This is equivalent to obtaining the systolic pressure, the diastolic pressure, and the like of the user.

In a possible implementation, the watch body is configured to: determine a plurality of pieces of transmural pressure of the radial artery based on the amplitudes of the plurality of pulse waves and a correspondence between an amplitude of a pulse wave and transmural pressure; and determine the transfer function of the radial artery for the pulse wave based on the plurality of pieces of transmural pressure. In the foregoing implementation, the processor may construct the feature vector of the pulse wave of the radial artery based on the amplitudes of the plurality of pulse waves of the radial artery, and then determine a feature vector of the transmural pressure of the radial artery based on a correspondence between an amplitude of a pulse wave and transmural pressure. The feature vector includes a plurality of elements, and the plurality of elements are a plurality of pieces of transmural pressure of the radial artery. After obtaining the feature vector of the transmural pressure of the radial artery, the processor may calculate the feature vector of the pulse wave of the radial artery, to obtain the transfer function of the radial artery for the pulse wave.

In a possible implementation, in the plurality of pulse waves of the radial artery, for any pulse wave, the pulse wave usually includes parts like a main wave, a tidal wave, and a dicrotic wave. Therefore, the amplitude of the pulse wave may include at least one of the following: an amplitude of the main wave of the pulse wave, an amplitude of the tidal wave of the pulse wave, and an amplitude of the dicrotic wave of the pulse wave.

In a possible implementation, a plurality of sensors may be disposed in the target area of the airbag, and the plurality of sensors form a sensor array. When the smartwatch is worn by the user on the wrist of the user, the sensor array may cover the radial artery of the wrist of the user. Therefore, each sensor in the sensor array may collect a plurality of pulse waves of the radial artery.

A second aspect of embodiments of this application provides a blood pressure measurement method. The method is implemented by using a smartwatch. The smartwatch includes a watch body, an airbag, and a sensor. The watch body is electrically connected to the sensor. The sensor is disposed on an inner wall of the airbag. When the smartwatch is worn on a wrist of a user, the airbag and the sensor cover a radial artery of the wrist. The method includes: measuring a plurality of pulse waves of the radial artery through the sensor when the airbag is under different pressure strength; and determining blood pressure of the user based on the plurality of pulse waves through the watch body.

The smartwatch configured to implement the foregoing method includes the watch body, the airbag, and the sensor. The watch body is electrically connected to the sensor. The sensor is disposed on the inner wall of the airbag. When the smartwatch is worn on the wrist of the user, the airbag and the sensor cover the radial artery of the wrist. Specifically, the watch body may first increase pressure strength of the airbag, so that the airbag squeezes the radial artery of the user. Therefore, the sensor may measure a plurality of pulse waves of the radial artery when the airbag is under different pressure strength. Then, the watch body may determine the blood pressure of the user based on the plurality of pulse waves of the radial artery. After the smartwatch is worn on the wrist of the user, the sensor in the airbag may directly cover the radial artery of the user, but does not cover an ulnar artery of the user. When the watch body inflates the airbag to make the airbag squeeze the radial artery of the user, the sensor may collect the plurality of pulse waves that are related only to the radial artery. Therefore, the blood pressure of the user determined by the watch body based on the plurality of pulse waves of the radial artery is accurate enough, and user experience can be improved.

In a possible implementation, a distance between a top of a target area of the airbag and a top of the airbag is greater than a first threshold. A length of the target area is greater than a second threshold. A width of the target area is less than a product of a width of the airbag and a preset coefficient. The target area is an area occupied by the sensor.

In a possible implementation, the method further includes: increasing the pressure strength of the airbag through the watch body, so that the airbag squeezes the radial artery until the plurality of pulse waves of the radial artery from the sensor meet a preset condition, where the plurality of pulse waves are in one-to-one correspondence with a plurality of pieces of pressure strength of the airbag.

In a possible implementation, the adjusting the pressure strength of the airbag through the watch body until the plurality of pulse waves of the radial artery from the sensor meet a preset condition includes: continuously increasing the pressure strength of the airbag through the watch body until an amplitude of a latest pulse wave in the plurality of pulse waves is less than a preset percentage of an amplitude of a target pulse wave, where the amplitude of the target pulse wave is the largest in amplitudes of the plurality of pulse waves; or intermittently increasing the pressure strength of the airbag through the watch body until an amplitude of a latest pulse wave in the plurality of pulse waves is less than an amplitude of a previous pulse wave; or continuously increasing the pressure strength of the airbag through the watch body until an amplitude of a latest pulse wave in the plurality of pulse waves is within a preset range.

In a possible implementation, the determining blood pressure of the user based on the plurality of pulse waves through the watch body includes: determining a transfer function of the radial artery for a pulse wave and a transfer function of skin of the user for a pulse wave based on the amplitudes of the plurality of pulse waves through the watch body; and calculating the plurality of pulse waves based on the transfer functions through the watch body, to obtain the blood pressure of the user.

In a possible implementation, the determining a transfer function of the radial artery for a pulse wave based on the amplitudes of the plurality of pulse waves through the watch body includes: determining a plurality of pieces of transmural pressure of the radial artery based on the amplitudes of the plurality of pulse waves and a correspondence between an amplitude of a pulse wave and transmural pressure through the watch body; and determining the transfer function of the radial artery for the pulse wave based on the plurality of pieces of transmural pressure through the watch body.

In a possible implementation, the amplitude of the pulse wave includes at least one of the following: an amplitude of a main wave of the pulse wave, an amplitude of a tidal wave of the pulse wave, and an amplitude of a dicrotic wave of the pulse wave.

In a possible implementation, there are a plurality of sensors, and the plurality of sensors form a sensor array.

A third aspect of embodiments of this application provides a processor. The processor is disposed in a watch body of a smartwatch. The smartwatch includes the watch body, an airbag, and a sensor. The watch body is electrically connected to the sensor. The sensor is disposed on an inner wall of the airbag. When the smartwatch is worn on a wrist of a user, the airbag and the sensor cover a radial artery of the wrist. The processor includes: an inflation module, configured to increase pressure strength of the airbag, so that the airbag squeezes the radial artery, and receives a plurality of pulse waves from the radial artery of the sensor; and a determining module, configured to determine blood pressure of the user based on the plurality of pulse waves.

The smartwatch in which the processor is located includes the watch body, the airbag, and the sensor. The watch body is electrically connected to the sensor. The sensor is disposed on the inner wall of the airbag. When the smartwatch is worn on the wrist of the user, the airbag and the sensor cover the radial artery of the wrist. Specifically, the watch body may first increase pressure strength of the airbag, so that the airbag squeezes the radial artery of the user. Therefore, the sensor may measure a plurality of pulse waves of the radial artery when the airbag is under different pressure strength. Then, the watch body may determine the blood pressure of the user based on the plurality of pulse waves of the radial artery. After the smartwatch is worn on the wrist of the user, the sensor in the airbag may directly cover the radial artery of the user, but does not cover an ulnar artery of the user. When the watch body inflates the airbag to make the airbag squeeze the radial artery of the user, the sensor may collect the plurality of pulse waves that are related only to the radial artery. Therefore, the blood pressure of the user determined by the watch body based on the plurality of pulse waves of the radial artery is accurate enough, and user experience can be improved.

In a possible implementation, a distance between a top of a target area of the airbag and a top of the airbag is greater than a first threshold. A length of the target area is greater than a second threshold. A width of the target area is less than a product of a width of the airbag and a preset coefficient. The target area is an area occupied by the sensor.

In a possible implementation, the inflation module is further configured to increase the pressure strength of the airbag, so that the airbag squeezes the radial artery until the plurality of pulse waves of the radial artery from the sensor meet a preset condition. The plurality of pulse waves are in one-to-one correspondence with a plurality of pieces of pressure strength of the airbag.

In a possible implementation, the inflation module is configured to: continuously increase the pressure strength of the airbag until an amplitude of a latest pulse wave in the plurality of pulse waves is less than a preset percentage of an amplitude of a target pulse wave, where the amplitude of the target pulse wave is the largest in amplitudes of the plurality of pulse waves; or intermittently increase the pressure strength of the airbag until an amplitude of a latest pulse wave in the plurality of pulse waves is less than an amplitude of a previous pulse wave; or continuously increase the pressure strength of the airbag until an amplitude of a latest pulse wave in the plurality of pulse waves is within a preset range.

In a possible implementation, the determining module is configured to: determine a transfer function of the radial artery for a pulse wave and a transfer function of skin of the user for a pulse wave based on the amplitudes of the plurality of pulse waves; and calculate the plurality of pulse waves based on the transfer functions, to obtain the blood pressure of the user.

In a possible implementation, the determining module is configured to: determine a plurality of pieces of transmural pressure of the radial artery based on the amplitudes of the plurality of pulse waves and a correspondence between an amplitude of a pulse wave and transmural pressure; and determine the transfer function of the radial artery for the pulse wave based on the plurality of pieces of transmural pressure.

In a possible implementation, the amplitude of the pulse wave includes at least one of the following: an amplitude of a main wave of the pulse wave, an amplitude of a tidal wave of the pulse wave, and an amplitude of a dicrotic wave of the pulse wave.

In a possible implementation, there are a plurality of sensors, and the plurality of sensors form a sensor array.

A fourth aspect of embodiments of this application provides a smartwatch. The smartwatch includes a memory and a processor. The memory stores code. The processor is configured to execute the code. When the code is executed, the smartwatch performs the method according to the second aspect or any one of the possible implementations of the second aspect.

A fifth aspect of embodiments of this application provides a computer storage medium. The computer storage medium stores one or more instructions. When the instructions are executed by one or more computers, the one or more computers are enabled to perform the method according to the second aspect or any one of the possible implementations of the second aspect.

A sixth aspect of embodiments of this application provides a computer program product. The computer program product stores instructions. When the instructions are executed by a computer, the computer is enabled to perform the method according to the second aspect or any one of the possible implementations of the second aspect.

A smartwatch provided in embodiments of this application includes a watch body, an airbag, and a sensor. The watch body is electrically connected to the sensor. The sensor is disposed on an inner wall of the airbag. When the smartwatch is worn on a wrist of a user, the airbag and the sensor cover a radial artery of the wrist. Specifically, the watch body may first increase pressure strength of the airbag, so that the airbag squeezes the radial artery of the user. Therefore, the sensor may measure a plurality of pulse waves of the radial artery when the airbag is under different pressure strength. Then, the watch body may determine blood pressure of the user based on the plurality of pulse waves of the radial artery. After the smartwatch is worn on the wrist of the user, the sensor in the airbag may directly cover the radial artery of the user, but does not cover an ulnar artery of the user. When the watch body inflates the airbag to make the airbag squeeze the radial artery of the user, the sensor may collect the plurality of pulse waves that are related only to the radial artery. Therefore, the blood pressure of the user determined by the watch body based on the plurality of pulse waves of the radial artery is accurate enough, and user experience can be improved.

**BRIEF DESCRIPTION OF DRAWINGS**

FIG. 1 is an example diagram of a structure of a watch body 100 of a smartwatch according to an embodiment of this application;

FIG. 2 is a diagram of a structure of a smartwatch according to an embodiment of this application;

FIG. 3 is a diagram of a structure of an airbag according to an embodiment of this application;

FIG. 4 is a diagram of a structure of a sensor array according to an embodiment of this application;

FIG. 5 is a diagram of another structure of a sensor array according to an embodiment of this application;

FIG. 6 is a diagram of still another structure of a sensor array according to an embodiment of this application;

FIG. 7 is a schematic flowchart of a blood pressure measurement method according to an embodiment of this application;

FIG. 8 is a diagram of inflating an airbag according to an embodiment of this application;

FIG. 9 is a diagram of an envelope signal according to an embodiment of this application;

FIG. 10 is another diagram of inflating an airbag according to an embodiment of this application;

FIG. 11 is another diagram of an envelope signal according to an embodiment of this application;

FIG. 12 is still another diagram of inflating an airbag according to an embodiment of this application;

FIG. 13 is a diagram of a pulse wave according to an embodiment of this application;

FIG. 14a is another diagram of a sensor array according to an embodiment of this application;

FIG. 14b is another diagram of a sensor array according to an embodiment of this application; and

FIG. 15 is a diagram of a structure of a processor according to an embodiment of this application.

**DESCRIPTION OF EMBODIMENTS**

Embodiments of this application provide a smartwatch and a blood pressure measurement method, so that a plurality of pulse waves of a radial artery of a user can be accurately obtained, and blood pressure of the user can be determined based on the plurality of pulse waves. Therefore, accuracy of blood pressure measurement can be improved, and user experience is further improved.

In the specification, claims, and the accompanying drawings of this application, the terms "first", "second", and the like are intended to distinguish similar objects but do not necessarily indicate a specific order or sequence. It should be understood that the terms used in such a way are interchangeable in appropriate circumstances. This is merely a discrimination manner that is used when objects having a same attribute are described in embodiments of this application. In addition, the terms "include", "contain" and any other variants mean to cover the non-exclusive inclusion, so that a process, method, system, product, or device that includes a series of units is not necessarily limited to those units, but may include other units not expressly listed or inherent to such a process, method, product, or device.

As a chronic disease with a largest number of patients in the world, hypertension is particularly harmful. To meet a requirement of a user for measuring blood pressure of the user anytime and anywhere, portable electronic devices like a smartwatch, a smart vest, and a smart band that have a blood pressure measurement function emerge.

Currently, an airbag may be integrated into the smartwatch, to improve convenience of blood pressure measurement. Specifically, the smartwatch usually includes a watch body, the airbag, and a sensor, and one end of the sensor is connected to one end of the airbag, and the other end of the sensor is connected to the watch body. When an air pump in the watch body inflates (pressurizes) the airbag, the airbag squeezes a wrist of the user. In this case, the sensor may collect a signal from the airbag, and send the collected signal to a processor in the watch body, so that the processor analyzes the signal, to obtain the blood pressure of the user.

However, the wrist usually includes a radial artery and an ulnar artery. Wrist sizes of some users are large, and the airbag may cover only the radial artery. In this case, signals collected by the sensor are all signals related to the radial artery. Wrist sizes of some other users are small, and the airbag may cover the radial artery and the ulnar artery. In this case signals collected by the sensor include both a signal related to the radial artery and a signal related to the ulnar artery, and signal types are inconsistent. As a result, finally obtained blood pressure of the user is inaccurate, and user experience is reduced.

Further, to prevent the wrist of the user from being excessively squeezed, an upper pressure limit of the airbag is usually set. As a result, the airbag can only enable the radial artery to reach a slightly squeezed state, but cannot enable the radial artery to reach another state. In this way, the sensor cannot collect signals that can represent various states of the radial artery, so that the finally obtained blood pressure of the user is inaccurate, and user experience is reduced.

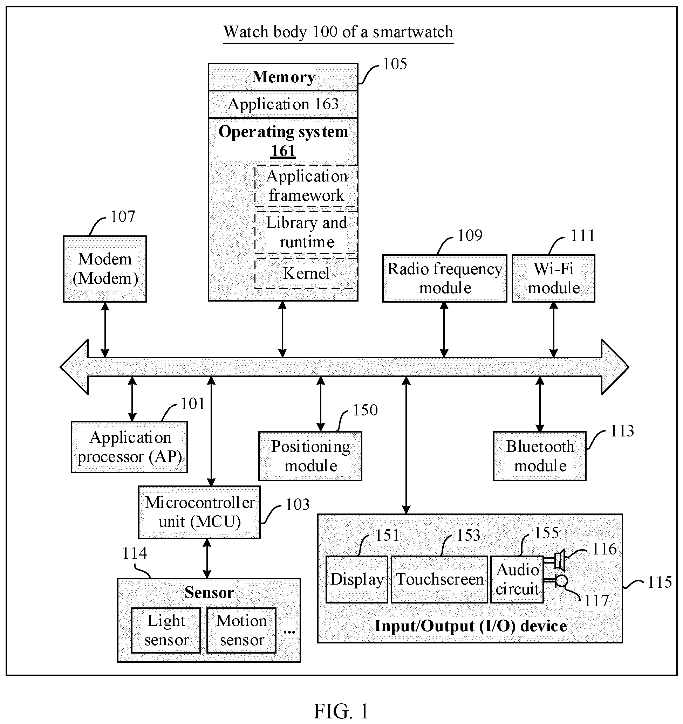

To resolve the foregoing method, an embodiment of this application provides a new type of smartwatch. The smartwatch may include components such as a watch body, a watchband, an airbag, and a sensor. The following first further describes the watch body of the smartwatch with reference to FIG. 1 (FIG. 1 is an example diagram of a structure of a watch body 100 of a smartwatch according to an embodiment of this application). As shown in FIG. 1, the watch body 100 of the smartwatch includes components such as an application processor 101, a microcontroller unit (microcontroller unit, MCU) 103, a memory 105, a modem (modem) 107, a radio frequency (radio frequency, RF) module 109, a wireless fidelity (Wireless-Fidelity, Wi-Fi for short) module 111, a Bluetooth module 113, a sensor 114, a positioning module 150, an input/output (input/output, I/O) device 115. These components can communicate with each other through one or more communication buses or signal cables. A person skilled in the art may understand that the hardware structure of shown in FIG. 1 does not constitute a limitation on the smartwatch. The watch body 100 may include more or fewer components than those shown in the figure, or may include a combination of some components, or may include different component arrangements.

The following specifically describes each part of the watch body 100 with reference to FIG. 1.

The application processor 101 is a control center of the watch body 100, and is connected to the components of the watch body 100 through various interfaces and buses. In some embodiments, the processor 101 may include one or more processing units.

The memory 105 stores a computer program, like an operating system 161 and an application 163 that are shown in FIG. 1. The application processor 101 is configured to execute the computer program in the memory 105, to implement a function defined by the computer program. For example, the application processor 101 executes the operating system 161 to implement various functions of the operating system on the watch body 100. The memory 105 further stores data other than the computer program, for example, data generated in running processes of the operating system 161 and the application 163. The memory 105 is a non-volatile storage medium, and generally includes an internal memory and an external memory. The internal memory includes, but is not limited to, a random access memory (Random Access Memory, RAM), a read-only memory (Read-Only Memory, ROM), a cache (cache), or the like. The external memory includes, but is not limited to, a flash memory (flash memory), a hard disk, a compact disc, a universal serial bus (universal serial bus, USB) flash drive, or the like. The computer program is generally stored in the external memory. Before executing a computer program, the processor loads the program from the external memory to the internal memory.

The memory 105 may be independent, and is connected to the application processor 101 by using a bus. Alternatively, the memory 105 and the application processor 101 may be integrated into a chip subsystem.

The MCU 103 is a coprocessor configured to obtain and process data from the sensor 114. A processing capability and power consumption of the MCU 103 are less than those of the application processor 101, but the MCU 103 has a feature of "always on (always on)", and can continuously collect and process the data from the sensor when the application processor 101 is in a sleep mode, to ensure normal running of the sensor with low power consumption. In an embodiment, the MCU 103 may be a sensor hub chip. The sensor 114 may include a light sensor and a motion sensor. Specifically, the light sensor may include an ambient light sensor and a proximity sensor. The ambient light sensor may adjust luminance of a display 151 based on brightness of ambient light. The proximity sensor may power off a display when the watch body 100 approaches an ear. As a type of motion sensor, an accelerometer sensor may detect values of accelerations in various directions (generally three axes), and may detect values and directions of gravity when the accelerometer sensor is still. The sensor 114 may further include another sensor like a gyroscope, a barometer, a hygrometer, a thermometer, or an infrared sensor. Details are not described herein. The MCU 103 and the sensor 114 may be integrated into a same chip, or may be separate components connected through a bus.

The modem 107 and the radio frequency module 109 constitute a communication subsystem of the watch body 100, and are configured to implement main functions of a wireless communication standard protocol like 3GPP or ETSI. The modem 107 is configured to perform encoding/decoding, signal modulation/demodulation, equalization, and the like. The radio frequency module 109 is configured to receive and send a radio signal, and the radio frequency module 109 includes but is not limited to an antenna, at least one amplifier, a coupler, a duplexer, and the like. The radio frequency module 109 cooperates with the modem 107 to implement a wireless communication function. The modem 107 may be used as an independent chip, or may be combined with another chip or circuit to form a system-level chip or an integrated circuit. These chips or integrated circuits may be applied to all smartwatches or the like that implement a wireless communication function.

The watch body 100 may further perform wireless communication via the Wi-Fi module 111, the Bluetooth module 113, and the like. The Wi-Fi module 111 is configured to provide, for the watch body 100, network access that complies with a Wi-Fi-related standard protocol. The watch body 100 may access a Wi-Fi access point via the Wi-Fi module 111, and further access the Internet. In some other embodiments, the Wi-Fi module 111 may alternatively be used as a Wi-Fi wireless access point, and may provide Wi-Fi network access for another electronic device. The Bluetooth module 113 is configured to implement short-range communication between the watch body 100 and another electronic device (for example, a mobile phone or a notebook computer). The Wi-Fi module 111 in this embodiment of this application may be an integrated circuit, a Wi-Fi chip, or the like, and the Bluetooth module 113 may be an integrated circuit, a Bluetooth chip, or the like.

The positioning module 150 is configured to determine a geographic location of the watch body 100. It may be understood that the positioning module 150 may be specifically a receiver of a positioning system like a global positioning system (global position system, GPS), a BeiDou navigation satellite system, or a Russian GLONASS.

The Wi-Fi module 111, the Bluetooth module 113, and the positioning module 150 may be independent chips or integrated circuits, or may be integrated together. For example, in an embodiment, the Wi-Fi module 111, the Bluetooth module 113, and the positioning module 150 may be integrated into a same chip. In another embodiment, the Wi-Fi module 111, the Bluetooth module 113, the positioning module 150, and the MCU 103 may alternatively be integrated into a same chip.

The input/output device 115 includes but is not limited to the display 151, a touchscreen 153, an audio circuit 155, and the like.

The touchscreen 153 may collect a touch event of a user of the watch body 100 on or near the touchscreen 153 (for example, an operation performed by the user on the touchscreen 153 or near the touchscreen 153 by using any suitable object like a finger or a stylus), and sends the collected touch event to another component (for example, the application processor 101). The operation performed by the user near the touchscreen 153 may be referred to as a floating touch. Through the floating touch, the user may select, move, or drag a target (for example, an icon) without directly touching the touchscreen 153. In addition, the touchscreen 153 may be implemented by using a plurality of types such as a resistive type, a capacitive type, an infrared type, and a surface acoustic wave type.

The display (also referred to as a display screen) 151 is configured to display information input by the user or information displayed to the user. The display may be configured in a form, for example, a liquid crystal display or an organic light-emitting diode. The touchscreen 153 may cover the display 151. After detecting a touch event, the touchscreen 153 transmits the touch event to the application processor 101 to determine a type of the touch event, and then the application processor 101 may provide corresponding visual output on the display 151 based on the type of the touch event. In FIG. 1, the touchscreen 153 and the display 151 are used as two independent components to implement input and output functions of the watch body 100. However, in some embodiments, the touchscreen 153 and the display 151 may be integrated to implement the input and output functions of the mobile phone 100. In addition, the touchscreen 153 and the display 151 may be disposed on a front side of the watch body 100 in a full panel form, to implement a bezel-less structure.

The audio circuit 155, a loudspeaker 116, and a microphone 117 may provide an audio interface between the user and the watch body 100. The audio circuit 109 may transmit an electrical signal converted from received audio data to the loudspeaker 113, and the loudspeaker 113 converts the electrical signal into a sound signal for output. In addition, the microphone 117 converts a collected sound signal into an electrical signal, and the audio circuit 109 receives the electrical signal, converts the electrical signal into audio data, and then sends the audio data to for example, another electronic device by using the modem 107 and the radio frequency module 109, or outputs the audio data to the memory 105 for further processing.

In addition, the watch body 100 may further have a fingerprint recognition function. For example, a fingerprint collection device may be configured on the back of the watch body 100 (for example, below a rear-facing camera), or a fingerprint collection device may be configured on the front of the watch body 100 (for example, below the touchscreen 153). For another example, a fingerprint collection component may be configured in the touchscreen 153 to implement the fingerprint recognition function. To be specific, the fingerprint collection component may be integrated with the touchscreen 153 to implement the fingerprint recognition function of the watch body 100. In this case, the fingerprint collection component is configured on the touchscreen 153, and may be a part of the touchscreen 153, or may be configured on the touchscreen 153 in another manner. A main part of the fingerprint collection component in this embodiment of this application is a fingerprint sensor. The fingerprint sensor may use any type of sensing technology, including but not limited to an optical sensing technology, a capacitive sensing technology, a piezoelectric sensing technology, an ultrasonic wave sensing technology, or the like.

Further, the operating system 161 used in the watch body 100 may be iOS®, Android®, Microsoft®, or another operating system. This is not limited in embodiments of this application.

The watch body 100 carrying the Android® operating system is used as an example. As shown in FIG. 1, the watch body 100 may be logically divided into a hardware layer, the operating system 161, and an application layer. The hardware layer includes hardware resources such as the hardware processor 101, the microcontroller unit 105, the modem 107, the Wi-Fi module 111, the sensor 114, and the positioning module 150 that are described above. The application layer includes one or more applications, for example, the application 163. The application 163 may be an application of any type, for example, a social application, an e-commerce application, or a browser. The operating system 161 is used as software middleware between the hardware layer and the application layer, and is a computer program for managing and controlling hardware and a software resource.

In an embodiment, the operating system 161 includes a kernel, a hardware abstraction layer (hardware abstraction layer, HAL), a library and runtime (library and runtime), and a framework (framework). The kernel is configured to provide an underlying system component and service, for example, power management, memory management, thread management, and a hardware driver. The hardware driver includes a Wi-Fi driver, a sensor driver, a positioning module driver, and the like. The hardware abstraction layer encapsulates a kernel driver, provides an interface to the framework, and shields an underlying implementation detail. The hardware abstraction layer 25 runs in user space, and the kernel driver runs in kernel space.

The library and runtime is also referred to as a runtime library, and provides a library file and an execution environment required by an executable program during running. In an embodiment, the library and runtime includes Android runtime (Android Runtime, ART), a library, and a scenario package runtime. The ART is a virtual machine or a virtual machine instance that can convert bytecode of an application into machine code. The library is a program library that provides support to the executable program during running, and includes a browser engine (like a webkit), a script execution engine (for example, a JavaScript engine), a graphics processing engine, and the like. The scenario package runtime is a running environment of a scenario package, and mainly includes a page execution environment (page context) and a script execution environment (script context). The page execution environment invokes a corresponding library to parse page code in an HTML, CSS, or another format, and the script execution environment invokes a corresponding function library to parse and execute code or an executable file implemented by a script language like JavaScript.

The framework is configured to provide various basic common components and services such as window management and location management for each application in the application layer. In an embodiment, the framework may include a geofence service, a policy service, a notification manager, and the like.

All functions of components in the operating system 161 described above may be implemented by the application processor 101 by executing the program stored in the memory 105.

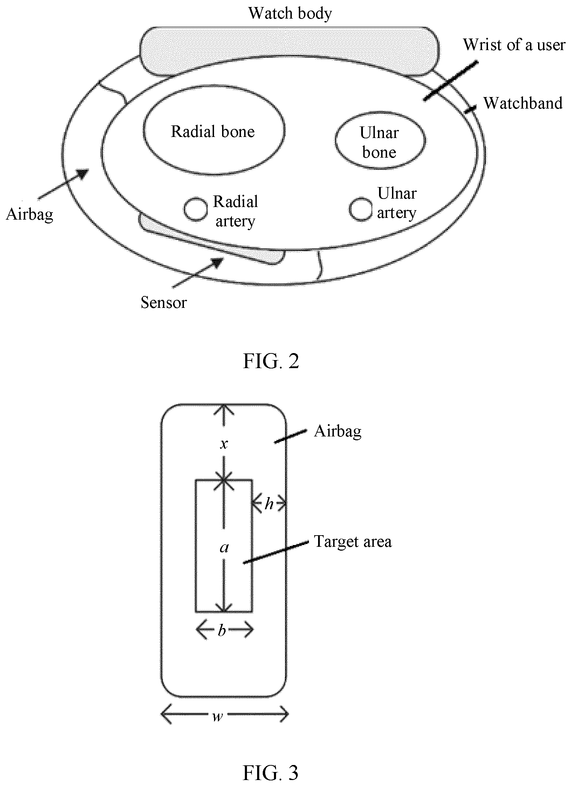

The foregoing describes in detail the internal structure of the watch body of the smartwatch, and the following describes an overall structure of the entire smartwatch. FIG. 2 is a diagram of a structure of a smartwatch according to an embodiment of this application. As shown in FIG. 2, the smartwatch includes a watch body and two watchbands. One end of one watchband is connected to one end of the watch body, and one end of the other watchband is connected to the other end of the watch body. At least a processor and an air pump are disposed inside the watch body. An airbag and a sensor are disposed inside one watchband.

In a cavity of the watchband, one end of the airbag is connected to one end of the watch body through a part of the watchband. Because the airbag is also provided with a cavity, the sensor may be disposed in the cavity of the airbag, the sensor is attached to an inner surface (namely, an inner wall) of the airbag, and the sensor is electrically connected to the processor. As shown in FIG. 2, when the smartwatch is worn by a user on a wrist of the user, the airbag and the sensor may cover a radial artery of the wrist of the user.

Specifically, for a user with a large wrist size, a user with a normal wrist size, and a user with a small wrist size, to enable the sensor to cover radial arteries of wrists of all the users as many as possible, and enable a pulse wave of the radial artery collected by the sensor to be an effective signal (that is, to enable the airbag part in which the sensor is located to effectively press-fit the radial artery), an area may be delimited on the inner surface of the airbag to place the sensor. This area is referred to as a target area, and a size of the target area may be set in the following manner.

In the airbag, a distance between one end of the target area (which may also be referred to as a top of the target area) and one end of the airbag (which may also be referred to as a top of the airbag) is greater than a first threshold (the first threshold may be set based on an actual requirement, and is not limited herein). A length of the target area is greater than a second threshold (the second threshold may be set based on an actual requirement, and is not limited herein). A width of the target area is less than a product of a width of the airbag and a preset coefficient (which may also be referred to as a force transfer attenuation coefficient of the airbag, where a value of the coefficient may be set based on an actual requirement, and is not limited herein).

For example, as shown in FIG. 3 (FIG. 3 is a diagram of a structure of the airbag according to an embodiment of this application), to ensure that the sensor can cover radial arteries of all users as many as possible (and the sensor does not cover ulnar arteries of the users), a large amount of wrist size data of the users is analyzed. The distance x between the top of the target area in the airbag and the top of the airbag may be greater than 25 mm, and the length a of the target area may be greater than 20 mm. To ensure that the pulse wave of the radial artery collected by the sensor is an effective signal, the airbag part in which the sensor is located needs to effectively press-fit the radial artery. The width b of the target area may be less than the width w of the airbag and the force transfer attenuation coefficient y of the airbag. A value of the force transfer attenuation coefficient y of the airbag is directly related to the structure of the airbag. For example, when a width w of a double-layer airbag is 30 mm, y is approximately equal to 0.7.

More specifically, there may be one or more sensors disposed in the target area, and the one or more sensors may form a sensor array. The sensor array may be one-dimensional or multidimensional. For example, the sensor array is a two-dimensional array. In this way, fidelity of signals collected by the sensors can be improved. When the smartwatch is worn by the user on the wrist of the user, the sensor array may cover the radial artery of the wrist of the user. Therefore, each sensor in the sensor array may collect a plurality of pulse waves of the radial artery.

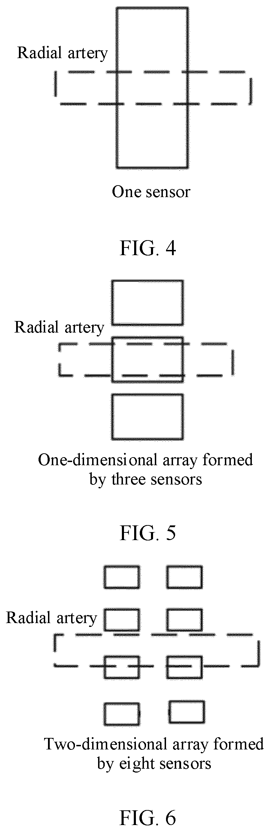

For example, refer to FIG. 4 to FIG. 6 (FIG. 4 is a diagram of a structure of the sensor array according to an embodiment of this application, FIG. 5 is a diagram of another structure of the sensor array according to an embodiment of this application, and FIG. 6 is a diagram of still another structure of the sensor array according to an embodiment of this application). FIG. 4 shows that one sensor is disposed in the target area of the airbag. FIG. 5 shows that three sensors are disposed in the target area of the airbag, and the three sensors form a one-dimensional array. FIG. 6 shows that eight sensors are disposed in the target area of the airbag, and the eight sensors form a two-dimensional array. Both a single sensor and a plurality of sensors can cover the radial artery of the user.

In this case, after the smartwatch is worn by the user on the wrist of the user, the user may deliver a blood pressure measurement instruction to the watch body of the smartwatch. The watch body may adjust pressure strength of the airbag based on the instruction, receive a plurality of pulse waves of the radial artery that are sent by the sensor, and then determine blood pressure of the user based on the plurality of pulse waves.

It should be noted that the foregoing example is merely described by using an example in which the airbag is disposed inside the watchband, and does not constitute a limitation on a location relationship between the airbag and the watchband. During actual application, the airbag may alternatively be disposed outside the watchband. In other words, the watchband is no longer integrally formed. Instead, the watchband is divided into two parts, and one end of the airbag is connected to one end of the watch body through a part of the watchband.

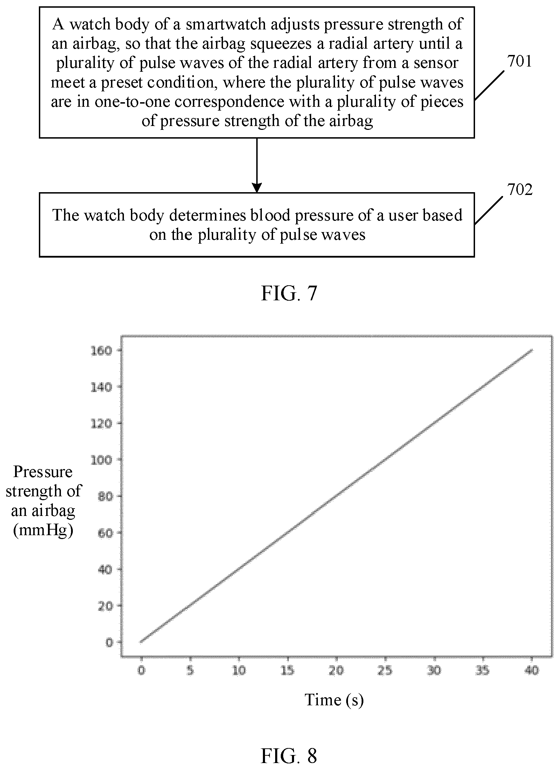

It should be further noted that the foregoing example is merely described by using an example in which the airbag is indirectly connected to the watch body. During actual application, the airbag may alternatively be directly connected to the watch body. In other words, the watchband is of a hollow structure, and the structure is used to place the airbag. In this case, one end of the airbag is directly connected to one end of the watch body. To further understand a working procedure of the smartwatch, the following further describes the working procedure with reference to FIG. 7. FIG. 7 is a schematic flowchart of a blood pressure measurement method according to an embodiment of this application. As shown in FIG. 7, the method may be implemented by using the smartwatch shown in FIG. 2. In the smartwatch, because working procedures of all sensors in the sensor array are similar, and similar processing is performed by the processor on signals sent by the sensors, the following uses one sensor for schematic description. The method includes the following steps.

701: The watch body of the smartwatch adjusts pressure strength of the airbag, so that the airbag squeezes a radial artery until a plurality of pulse waves of the radial artery from the sensor meet a preset condition, where the plurality of pulse waves are in one-to-one correspondence with a plurality of pieces of pressure strength of the airbag.

In this embodiment, after receiving a blood pressure measurement instruction delivered by the user, the processor in the watch body may control, based on the instruction, the air pump in the watch body to continuously increase the pressure strength inside the airbag (that is, inflate the airbag a plurality of times), to obtain the plurality of pieces of pressure strength of the airbag. The airbag under the plurality of pieces of pressure strength may continuously squeeze the radial artery of the wrist of the user. In this process, the sensor may detect the radial artery, to obtain the plurality of pulse waves of the radial artery, and then send the plurality of pulse waves to the processor. The plurality of pulse waves of the radial artery are in one-to-one correspondence with the plurality of pieces of pressure strength of the airbag. After determining that the plurality of pulse waves of the radial artery meet the preset condition, the processor no longer increases the pressure strength of the airbag (that is, no longer inflates the airbag).

Specifically, at a first moment, after increasing original pressure strength of the airbag (that is, inflating the airbag for a first time), the processor may obtain first pressure strength of the airbag, the airbag under the first pressure strength may squeeze the radial artery of the user to a degree, and the sensor may obtain a first pulse wave of the squeezed radial artery, and send the first pulse wave to the processor; at a second moment, after increasing the first pressure strength of the airbag (that is, inflating the airbag for a second time), the processor may obtain second pressure strength of the airbag, the airbag under the second pressure strength may squeeze the radial artery of the user to another degree, and the sensor may obtain a second pulse wave of the squeezed radial artery, and send the second pulse wave to the processor; ...; and at a current moment, after increasing previous pressure strength of the airbag (that is, performing latest inflating on the airbag), the processor obtains latest pressure strength of the airbag, the airbag under the latest pressure strength may squeeze the radial artery of the user to a latest degree, and the sensor may obtain a latest pulse wave of the squeezed radial artery, and sends the latest pulse wave to the processor. It can be learned that the processor may continuously increase the original pressure strength of the airbag a plurality of times, to successively obtain the plurality of pieces of pressure strength of the airbag (including the first pressure strength, the second pressure strength, ..., and the latest pressure strength of the airbag). In addition, the sensor may successively collect the plurality of pulse waves of the radial artery (including the first pulse wave, the second pulse wave, ..., and the latest pulse wave of the radial artery), and send the plurality of pulse waves to the processor in time.

It should be noted that, after obtaining the latest pulse wave, the processor may determine whether the latest pulse wave meets the preset condition (each time the processor obtains a pulse wave, the processor performs a determining operation). If the latest pulse wave meets the preset condition, the processor no longer inflates the airbag. If the latest pulse wave does not meet the preset condition, the processor continues to inflate the airbag until an updated pulse wave meets the preset condition.

More specifically, the processor may determine, in a plurality of manners, whether the latest pulse wave meets the preset condition.

(1) The processor may continuously (that is, the first moment, the second moment, ..., and the latest moment are all consecutive moments) increase the pressure strength of the airbag, that is, continuously inflate the airbag. If an amplitude of the latest pulse wave from the sensor is less than a preset percentage of an amplitude of a target pulse wave (a value of the percentage may be set based on an actual requirement, and is not limited herein), it indicates that the radial artery of the user is in a closed state, and the processor deflates the airbag. It should be noted that the target pulse wave is a pulse wave with a largest amplitude in the plurality of pulse waves collected by the sensor, and the target pulse wave is usually a pulse wave previous to the latest pulse wave.

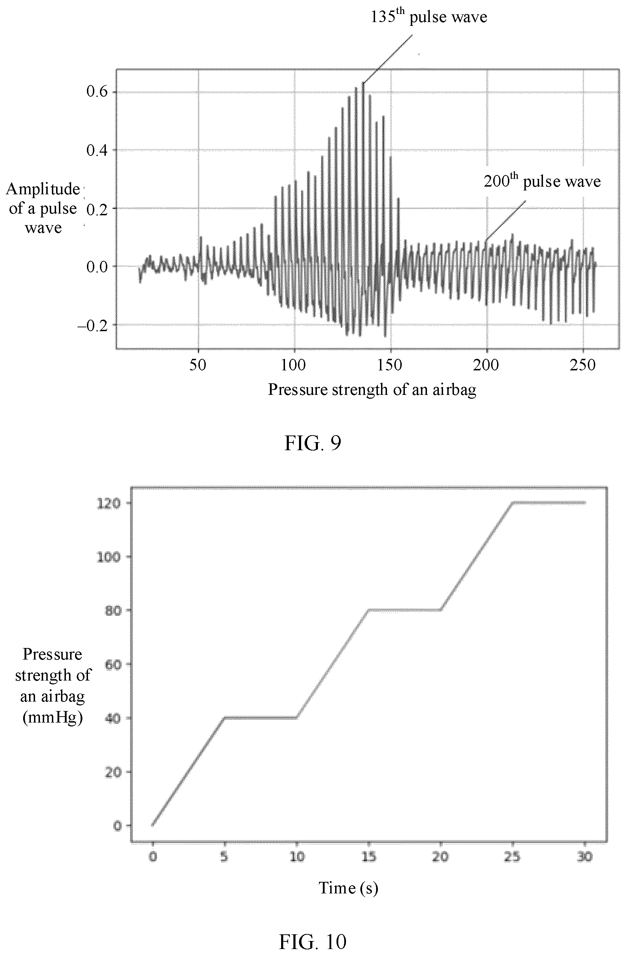

For example, refer to FIG. 8 and FIG. 9 (FIG. 8 is a diagram of inflating the airbag according to an embodiment of this application, and FIG. 9 is a diagram of an envelope signal according to an embodiment of this application). It is assumed that the original pressure strength of the airbag is 0, and at a moment of 0s, the processor may perform linear (continuous) pressurization on the airbag at 4 mmHg/s. In this case, at a moment of 1s, pressure strength of the airbag is 4 mmHg; at a moment of 2s, pressure strength of the airbag is 8 mmHg; ...; at a moment of 50s, pressure strength of the airbag is 200 mmHg; and so on. In a pressurization process, when the pressure strength of the airbag is 1 mmHg, after the airbag with the pressure strength of 1 mmHg squeezes the radial artery of the user, the sensor may send a collected first pulse wave of the radial artery to the processor; when the pressure strength of the airbag is 2 mmHg, after the airbag with the pressure strength of 2 mmHg squeezes the radial artery of the user, the sensor may send a collected second pulse wave of the radial artery to the processor; ...; and when the pressure strength of the airbag is 200 mmHg, after the airbag with the pressure strength of 200 mmHg squeezes the radial artery of the user, the sensor may send a collected 200th pulse wave of the radial artery to the processor. It is assumed that the moment of 50s is the current moment, and the 200th pulse wave is the latest pulse wave. As shown in FIG. 9, an amplitude of the 200th pulse wave is 0.061, and a 135th pulse wave has a largest amplitude, which is 0.63. The processor may determine that the amplitude of the 200th pulse wave is just 10% less than the amplitude of the 135th pulse wave (certainly, the processor also performs a similar determining operation on the first pulse wave to a 199th pulse wave, but none of these pulse waves meets the condition, which indicates that the radial artery is not in the closed state). In this case, it indicates that the radial artery is in the closed state. After the moment of 50s, the processor no longer pressurizes the airbag, that is, depressurizes the airbag until the pressure strength of the airbag is 0.

It should be noted that, in the examples shown in FIG. 8 and FIG. 9, an example in which continuous pressurization is linear pressurization is merely used for description. During actual application, the continuous pressurization may alternatively be non-linear pressurization. In other words, at different moments, the processor pressurizes the airbag at different pressurization speeds. For example, at the moment of 0s, the processor may pressurize the airbag at 4 mmHg/s, and at the moment of 1s, the processor may pressurize the airbag at 5 mmHg/s.

(2) The processor may intermittently (that is, the first moment, the second moment, ..., and the latest moment may be consecutive moments, or may be non-consecutive moments) increase the pressure strength of the airbag, that is, first inflate the airbag and keep the airbag neither inflated nor deflated, then continue to inflate the airbag and keep the airbag neither inflated nor deflated, and so on. If an amplitude of the latest pulse wave is less than an amplitude of a previous pulse wave, it indicates that the radial artery of the user is in a flat state, and the processor deflates the airbag.

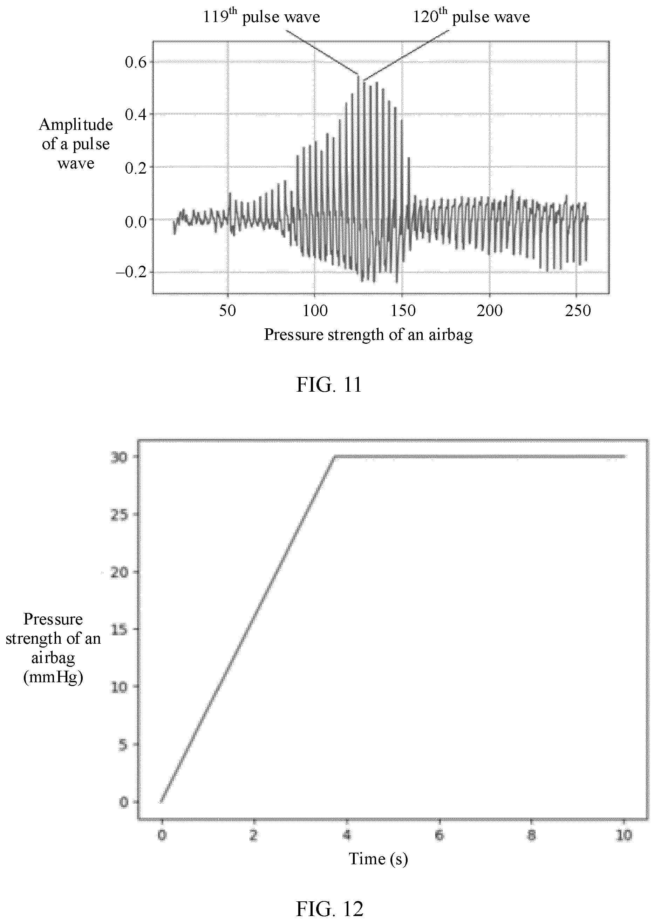

For example, refer to FIG. 10 and FIG. 11 (FIG. 10 is another diagram of inflating the airbag according to an embodiment of this application, and FIG. 11 is another diagram of an envelope signal according to an embodiment of this application). It is assumed that the original pressure strength of the airbag is 0, and at a moment of 0s, the processor may perform multi-segment stepped (intermittent) pressurization on the airbag at 4 mmHg/s. In this case, at a moment of 1s, pressure strength of the airbag is 4 mmHg; at a moment of 2s, pressure strength of the airbag is 8 mmHg; ...; at a moment of 5s, pressure strength of the airbag is 40 mmHg; during a period from 5s to 10s, pressure strength of the airbag remains 40 mmHg; at a moment of 11s, pressure strength of the airbag is 44 mmHg; and so on. In a pressurization process, when the pressure strength of the airbag is 1 mmHg, after the airbag with the pressure strength of 1 mmHg squeezes the radial artery of the user, the sensor may send a collected first pulse wave of the radial artery to the processor; when the pressure strength of the airbag is 2 mmHg, after the airbag with the pressure strength of 2 mmHg squeezes the radial artery of the user, the sensor may send a collected second pulse wave of the radial artery to the processor; ...; and when the pressure strength of the airbag is 120 mmHg, after the airbag with the pressure strength of 120 mmHg squeezes the radial artery of the user, the sensor may send a collected 120th pulse wave of the radial artery to the processor. It is assumed that a moment of 25s is the current moment, and the 120th pulse wave is the latest pulse wave. As shown in FIG. 9, an amplitude of the 120th pulse wave is 0.58, and a 119th pulse wave has a largest amplitude, which is 0.59. The processor may determine that the amplitude of the 120th pulse wave is just less than the amplitude of the 119th pulse wave (certainly, the processor also performs a similar determining operation on the first pulse wave to the 119th pulse wave, but none of these pulse waves meets the condition, which indicates that the radial artery is not in the flat state). In this case, it indicates that the radial artery is in the flat state. After the moment of 25s, the processor no longer pressurizes the airbag. In other words, in a period from 25s to 30s, the processor maintains the pressure strength of the airbag at 120 mmHg. After the moment of 30s, the processor depressurizes the airbag until the pressure strength of the airbag is 0.

(3) The processor may continuously (that is, the first moment, the second moment, ..., and the latest moment are all consecutive moments) increase the pressure strength of the airbag, that is, continuously inflate the airbag. If an amplitude of the latest pulse wave from the sensor is within a preset range (the range may be set based on an actual requirement, and is not limited herein), it indicates that the radial artery of the user is in a slightly squeezed state, and the processor neither inflates nor deflates the airbag.

For example, refer to FIG. 12 (FIG. 12 is still another diagram of inflating the airbag according to an embodiment of this application). It is assumed that the original pressure strength of the airbag is 0, and at a moment of 0s, the processor may perform linear (continuous) pressurization on the airbag at 7.5 mmHg/s. In this case, at a moment of 1s, pressure strength of the airbag is 7.5 mmHg; at a moment of 2s, pressure strength of the airbag is 15 mmHg; ...; at a moment of 4s, pressure strength of the airbag is 30 mmHg; and so on. In a pressurization process, when the pressure strength of the airbag is 1 mmHg, after the airbag with the pressure strength of 1 mmHg squeezes the radial artery of the user, the sensor may send a collected first pulse wave of the radial artery to the processor; when the pressure strength of the airbag is 2 mmHg, after the airbag with the pressure strength of 2 mmHg squeezes the radial artery of the user, the sensor may send a collected second pulse wave of the radial artery to the processor; ...; and when the pressure strength of the airbag is 30 mmHg, after the airbag with the pressure strength of 30 mmHg squeezes the radial artery of the user, the sensor may send a collected 30th pulse wave of the radial artery to the processor. It is assumed that the moment of 4s is the current moment, and an amplitude of the 30th pulse wave is 0.05, which is within a range of 0.05 to 0.06. In this case, it indicates that the radial artery is in the slightly squeezed state. After the moment of 4s, the processor maintains the pressure strength of the airbag at 30 mmHg.

702: The watch body determines blood pressure of the user based on the plurality of pulse waves.

After obtaining the plurality of pulse waves of the radial artery that are sent by the sensor, the processor in the watch body may process the plurality of pulse waves, to obtain the blood pressure of the user, for example, systolic pressure and diastolic pressure of the user.

Specifically, the processor may obtain the blood pressure of the user in the following manners.

(1) The processor may construct a feature vector (where the feature vector may indicate a waveform change of the pulse wave of the radial artery, the feature vector includes a plurality of elements, and the plurality of elements are the amplitudes of the plurality of pulse waves of the radial artery) based on the amplitudes of the plurality of pulse waves of the radial artery. Then, the processor determines, based on the feature vector of the pulse wave of the radial artery, a transfer function of the radial artery of the user for a pulse wave and a transfer function of skin of the user for a pulse wave, namely, an effect of the radial artery on the pulse wave and an effect of the skin on the pulse wave.

More specifically, the processor may obtain the transfer function of the radial artery for the pulse wave and the transfer function of the skin for the pulse wave in the following manners.

(1.1) The processor may construct the feature vector of the pulse wave of the radial artery based on the amplitudes of the plurality of pulse waves of the radial artery, and then determine a feature vector of transmural pressure of the radial artery based on a correspondence between an amplitude of a pulse wave and transmural pressure (where the feature vector may represent a change of the transmural pressure of the radial artery, the feature vector includes a plurality of elements, and the plurality of elements are a plurality of pieces of transmural pressure of the radial artery). It should be noted that, in this embodiment, the transmural pressure of the radial artery usually refers to a difference between the pressure strength of the airbag and the blood pressure of the user (namely, pressure strength inside the radial artery).

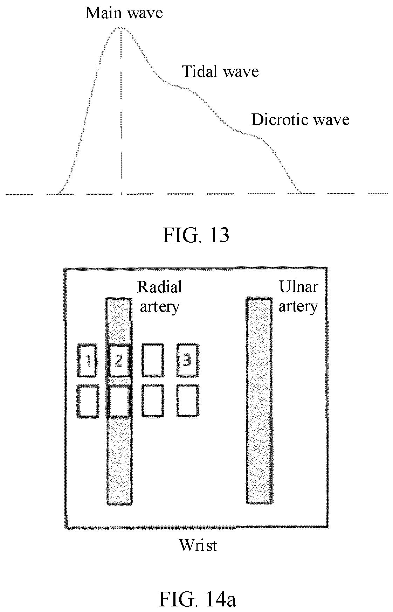

It should be noted that, as shown in FIG. 13 (FIG. 13 is a diagram of a pulse wave according to an embodiment of this application), in the plurality of pulse waves of the radial artery, for any pulse wave, the pulse wave usually includes parts like a main wave, a tidal wave, and a dicrotic wave. Therefore, an amplitude of the pulse wave may include at least one of the following: an amplitude of the main wave of the pulse wave, an amplitude of the tidal wave of the pulse wave, and an amplitude of the dicrotic wave of the pulse wave. This is not limited herein.

(1.2) After obtaining the feature vector of the transmural pressure of the radial artery, the processor may calculate the feature vector of the pulse wave of the radial artery, to obtain the transfer function of the radial artery for the pulse wave.

(1.3) After obtaining the feature vector of the pulse wave of the radial artery, the processor may calculate the feature vector of the pulse wave of the radial artery, to obtain the transfer function of the skin for the pulse wave.

Still refer to the examples shown in FIG. 8 and FIG. 9. After the processor obtains the first pulse wave to the 200th pulse wave of the radial artery, a waveform change of the pulse wave of the radial artery may be determined based on the amplitudes of the 200 pulse waves, and a change of the transmural pressure of the radial artery may be determined based on the waveform change of the pulse wave of the radial artery. Then, the processor may determine the transfer function $J_{artery}$ of the radial artery for the pulse wave based on the change of the transmural pressure of the radial artery. Subsequently, the processor may further determine the transfer function $f_{skin}$ of the skin for the pulse wave based on the waveform change of the pulse wave of the radial artery.

It should be noted that, if the examples shown in FIG. 10 and FIG. 11 are used, the processor obtains the transfer function $J_{artery}$ of the radial artery for the pulse wave and the transfer function $f_{skin}$ of the skin for the pulse wave based on the amplitudes of the first pulse wave to the 120th pulse wave of the radial artery. If the example shown in FIG. 12 is used, the processor obtains the transfer function $J_{artery}$ of the radial artery for the pulse wave and the transfer function $f_{skin}$ of the skin for the pulse wave based on the amplitudes of the first pulse wave to the 30th pulse wave of the radial artery.

(2) After obtaining the transfer function of the radial artery for the pulse wave and the transfer function of the skin for the pulse wave, the processor may calculate the plurality of pulse waves based on the transfer function of the radial artery for the pulse wave and the transfer function of the skin for the pulse wave, to obtain the blood pressure of the user. This is equivalent to obtaining the systolic pressure, the diastolic pressure, and the like of the user.

The foregoing examples are still used. The transfer function $f_{artery}$ of the radial artery for the pulse wave and the transfer function $f_{skin}$ of the skin for the pulse wave are obtained, and the blood pressure (wave) of the user may be calculated according to the following formula:

$$P = f_{skin}\left(f_{artery}\left(P_{pulse}\right)\right) \quad (1)$$

In the foregoing formula, $P$ is an envelope signal, the envelope signal includes the plurality of pulse waves sent by the sensor, and $P_{pulse}$ is a blood pressure wave of the user.

In addition, in the foregoing embodiments, only a sensor in the smartwatch is used as an example for description. If the smartwatch further includes another sensor, the processor may also perform step 701 and step 702 on a plurality of pulse waves collected by the another sensor. In other words, for a plurality of pulse waves of the radial artery collected by one of a plurality of sensors, the processor may calculate one piece of blood pressure of the user. This is also applicable to other sensors. Therefore, the processor may finally obtain a plurality of pieces of blood pressure of the user. Generally, if the plurality of pieces of blood pressure are usually similar, the processor may randomly select one as the final blood pressure of the user. If the plurality of pieces of blood pressure differ greatly, the processor may perform weighted summation on the plurality of pieces of blood pressure, to obtain the final blood pressure of the user.

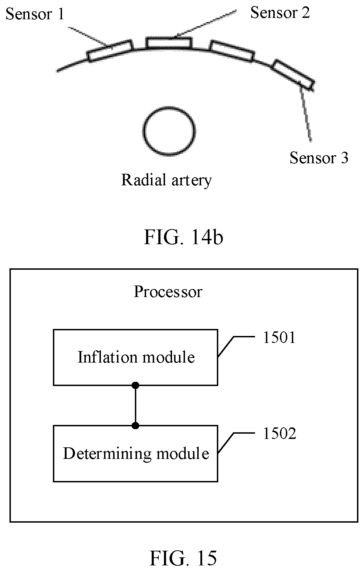

For example, refer to FIG. 14a and FIG. 14b (FIG. 14a is another diagram of a sensor array according to an embodiment of this application, and FIG. 14b is another diagram of a sensor array according to an embodiment of this application). It is assumed that the sensor array includes eight sensors and is a two-dimensional array, and sensors 1, 2, and 3 are used as examples for description. After the processor receives a plurality of pulse waves $P_1$ of the radial artery sent by the sensor 1, a plurality of pulse waves $P_2$ of the radial artery sent by the sensor 2, and a plurality of pulse waves $P_3$ of the radial artery sent by the sensor 3 (in this case, it is assumed that the plurality of pulse waves collected by the three sensors all meet the preset condition, and the processor no longer inflates the airbag), the processor may determine, based on the plurality of pulse waves $P_1$ of the radial artery sent by the sensor 1, a transfer function $f_{artery}$ of the radial artery for the pulse wave and a transfer

function $f_{skin1}$ of skin on which the sensor 1 is located for the pulse wave. Similarly, the processor may determine, based on the plurality of pulse waves $P_2$ of the radial artery sent by the sensor 2, a transfer function $J_{artery}$ of the radial artery for the pulse wave and a transfer function $f_{san2}$ of skin on which the sensor 2 is located for the pulse wave. The processor may determine, based on the plurality of pulse waves $P_3$ of the radial artery sent by the sensor 3, a transfer function $f_{artery}$ of the radial artery for the pulse wave and a transfer function $f_{skin3}$ of skin on which the sensor 3 is located for the pulse wave. Specifically, the processor may obtain the blood pressure wave $P_{pulse}$ of the user according to the following formula:

$$P_1 = f_{skin1}(f_{artery}(P_{pulse}))$$
$$P_2 = f_{skin2}(f_{artery}(P_{pulse})) \quad (2)$$
$$P_3 = f_{skin3}(f_{artery}(P_{pulse}))$$

After obtaining the blood pressure wave of the user, the processor may display the diastolic pressure and the systolic pressure of the user on the display of the watch body, so that the user can watch and use the diastolic pressure and the systolic pressure, and a blood pressure measurement requirement of the user is met.

The smartwatch provided in this embodiment of this application includes the watch body, the airbag, and the sensor. The sensor is disposed inside the airbag. The sensor is attached to a surface of the airbag. When the smartwatch is worn on the wrist of the user, the airbag and the sensor cover the radial artery of the wrist of the user. Specifically, the watch body may first increase the pressure strength of the airbag, so that the airbag squeezes the radial artery until the plurality of pulse waves of the radial artery from the sensor meet the preset condition. The plurality of pulse waves of the radial artery are in one-to-one correspondence with the plurality of pieces of pressure strength of the airbag. Then, the watch body may determine the blood pressure of the user based on the plurality of pulse waves of the radial artery. After the smartwatch is worn on the wrist of the user, the sensor in the airbag may directly cover the radial artery of the user, but does not cover an ulnar artery of the user. When the watch body inflates the airbag to make the airbag squeeze the radial artery of the user, the sensor may collect the plurality of pulse waves that are related only to the radial artery. Therefore, the blood pressure of the user determined by the watch body based on the plurality of pulse waves of the radial artery is accurate enough, and user experience can be improved.

Further, in the smartwatch provided in this embodiment of this application, the watch body may inflate the airbag in a plurality of manners (for example, continuous pressurization and intermittent pressurization), and a condition for stopping inflation is set. In this way, an upper pressure limit of the airbag can be flexibly adjusted, and comfort of the user is ensured. In addition, the adjustable pressure upper limit may enable the radial artery of the user to reach a plurality of states like the slightly squeezed state, the flat state, and the closed state, and the sensor may collect signals that can represent various states of the radial artery. In this way, the finally obtained blood pressure of the user is accurate enough, and user experience can be improved.

The foregoing describes in detail the blood pressure measurement method provided in embodiments of this application. The following describes a processor provided in embodiments of this application. FIG. 15 is a diagram of a structure of a processor according to an embodiment of this application. As shown in FIG. 15, the processor is disposed in a watch body of a smartwatch. The smartwatch includes the watch body, an airbag, and a sensor. The watch body is electrically connected to the sensor. The sensor is disposed on an inner wall of the airbag. When the smartwatch is worn on a wrist of a user, the airbag and the sensor cover a radial artery of the wrist. Specifically, the processor includes:

an inflation module 1501, configured to increase pressure strength of the airbag, so that the airbag squeezes the radial artery, and receives a plurality of pulse waves from the radial artery of the sensor; and a determining module 1502, configured to determine blood pressure of the user based on the plurality of pulse waves.

A smartwatch provided in embodiments of this application includes a watch body, an airbag, and a sensor. The watch body is electrically connected to the sensor. The sensor is disposed on an inner wall of the airbag. When the smartwatch is worn on a wrist of a user, the airbag and the sensor cover a radial artery of the wrist. Specifically, the watch body may first increase pressure strength of the airbag, so that the airbag squeezes the radial artery of the user. Therefore, the sensor may measure a plurality of pulse waves of the radial artery when the airbag is under different pressure strength. Then, the watch body may determine blood pressure of the user based on the plurality of pulse waves of the radial artery. After the smartwatch is worn on the wrist of the user, the sensor in the airbag may directly cover the radial artery of the user, but does not cover an ulnar artery of the user. When the watch body inflates the airbag to make the airbag squeeze the radial artery of the user, the sensor may collect the plurality of pulse waves that are related only to the radial artery. Therefore, the blood pressure of the user determined by the watch body based on the plurality of pulse waves of the radial artery is accurate enough, and user experience can be improved.

In a possible implementation, a distance between a top of a target area of the airbag and a top of the airbag is greater than a first threshold. A length of the target area is greater than a second threshold. A width of the target area is less than a product of a width of the airbag and a preset coefficient. The target area is an area occupied by the sensor.

In a possible implementation, the inflation module 1501 is further configured to increase the pressure strength of the airbag, so that the airbag squeezes the radial artery until the plurality of pulse waves of the radial artery from the sensor meet a preset condition. The plurality of pulse waves are in one-to-one correspondence with a plurality of pieces of pressure strength of the airbag.

In a possible implementation, the inflation module 1501 is configured to: continuously increase the pressure strength of the airbag until an amplitude of a latest pulse wave in the plurality of pulse waves is less than a preset percentage of an amplitude of a target pulse wave, where the amplitude of the target pulse wave is the largest in amplitudes of the plurality of pulse waves; or intermittently increase the pressure strength of the airbag until an amplitude of a latest pulse wave in the plurality of pulse waves is less than an amplitude of a previous pulse wave; or continuously increase the pressure strength of the airbag until an amplitude of a latest pulse wave in the plurality of pulse waves is within a preset range.

In a possible implementation, the determining module 1502 is configured to: determine a transfer function of the radial artery for a pulse wave and a transfer function of skin of the user for a pulse wave based on the amplitudes of the plurality of pulse waves; and calculate the plurality of pulse waves based on the transfer functions, to obtain the blood pressure of the user.

In a possible implementation, the determining module 1502 is configured to: determine a plurality of pieces of transmural pressure of the radial artery based on the amplitudes of the plurality of pulse waves and a correspondence between an amplitude of a pulse wave and transmural pressure; and determine the transfer function of the radial artery for the pulse wave based on the plurality of pieces of transmural pressure.

In a possible implementation, the amplitude of the pulse wave includes at least one of the following: an amplitude of a main wave of the pulse wave, an amplitude of a tidal wave of the pulse wave, and an amplitude of a dicrotic wave of the pulse wave.

In a possible implementation, there are a plurality of sensors, and the plurality of sensors form a sensor array.

It should be noted that, content such as information exchange between the modules/units of the apparatuses and an execution process is based on the same concept as the method embodiments of this application, and produces the same technical effects as those of the method embodiments of this application. For specific content, refer to the foregoing descriptions in the method embodiments of this application. Details are not described herein again.

An embodiment of this application further relates to a computer storage medium. The computer-readable storage medium stores a program used for signal processing. When the program runs on a computer, the computer is enabled to perform the steps performed by the processor in the embodiment shown in FIG. 7.

An embodiment of this application further relates to a computer program product. The computer program product stores instructions, and when the instructions are executed by a computer, the computer is enabled to perform the steps performed by the processor in the embodiment shown in FIG. 7.

It may be clearly understood by a person skilled in the art that, for the purpose of convenient and brief description, for a detailed working process of the foregoing system, apparatus, and unit, refer to a corresponding process in the foregoing method embodiments, and details are not described herein again.

In the several embodiments provided in this application, it should be understood that the disclosed system, apparatus, and method may be implemented in other manners. For example, the described apparatus embodiment is merely an example. For example, division into units is merely logical function division and may be other division in an actual implementation. For example, a plurality of units or components may be combined or integrated into another system, or some features may be ignored or not performed. In addition, the displayed or discussed mutual couplings or direct couplings or communication connections may be implemented through some interfaces. The indirect couplings or communication connections between the apparatuses or units may be implemented in electrical, mechanical, or other forms.

The units described as separate components may or may not be physically separate, and parts displayed as units may or may not be physical units, may be located in one place, or may be distributed on a plurality of network units. Some or all of the units may be selected based on actual requirements to achieve the objectives of the solutions of embodiments.

In addition, function units in embodiments of this application may be integrated into one processing unit, or each of the units may exist alone physically, or two or more units may be integrated into one unit. The integrated unit may be implemented in a form of hardware, or may be implemented in a form of a software function unit.

When the integrated unit is implemented in a form of a software function unit and sold or used as an independent product, the integrated unit may be stored in a computer-readable storage medium. Based on such an understanding, the technical solutions of this application essentially, or the part contributing to the conventional technology, or all or some of the technical solutions may be implemented in a form of a software product. The computer software product is stored in a storage medium, and includes several instructions for instructing a computer device (which may be a personal computer, a server, or a network device) to perform all or some of the steps of the methods described in embodiments of this application. The foregoing storage medium includes any medium that can store program code, such as a USB flash drive, a removable hard disk, a read-only memory (read-only memory, ROM), a random access memory (random access memory, RAM), a magnetic disk, or a compact disc.

**Claims**

1. A smartwatch, wherein the smartwatch comprises a watch body, an airbag, and a sensor, the watch body is electrically connected to the sensor, the sensor is disposed on an inner wall of the airbag, and when the smartwatch is worn on a wrist of a user, the airbag and the sensor cover a radial artery of the wrist;

   the sensor is configured to measure a plurality of pulse waves of the radial artery when the airbag is under different pressure strength;
   the watch body is configured to determine blood pressure of the user based on the plurality of pulse waves.

2. The smartwatch according to claim 1, wherein a distance between a top of a target area of the airbag and a top of the airbag is greater than a first threshold, a length of the target area is greater than a second threshold, a width of the target area is less than a product of a width of the airbag and a preset coefficient, and the target area is an area occupied by the sensor.

3. The smartwatch according to claim 1 or 2, wherein the watch body is further configured to increase pressure strength of the airbag, so that the airbag squeezes the radial artery until the plurality of pulse waves of the radial artery from the sensor meet a preset condition, wherein the plurality of pulse waves are in one-to-one correspondence with a plurality of pieces of pressure strength of the airbag.

4. The smartwatch according to claim 3, wherein the watch body is configured to:

   continuously increase the pressure strength of the airbag until an amplitude of a latest pulse wave in the plurality of pulse waves is less than a preset percentage of an amplitude of a target pulse wave, wherein the amplitude of the target pulse wave is the largest in amplitudes of the plurality of pulse waves; or
   intermittently increase the pressure strength of the airbag until an amplitude of a latest pulse wave in the plurality of pulse waves is less than an amplitude of a previous pulse wave; or
   continuously increase the pressure strength of the airbag until an amplitude of a latest pulse wave in the plurality of pulse waves is within a preset range.

5. The smartwatch according to any one of claims 1 to 4, wherein the watch body is configured to:

   determine a transfer function of the radial artery for a pulse wave and a transfer function of skin of the user for a pulse wave based on the amplitudes of the plurality of pulse waves; and calculate the plurality of pulse waves based on the transfer functions, to obtain the blood pressure of the user.

6. The smartwatch according to claim 5, wherein the watch body is configured to:

   determine a plurality of pieces of transmural pressure of the radial artery based on the amplitudes of the plurality of pulse waves and a correspondence between an amplitude of a pulse wave and transmural pressure; and determine the transfer function of the radial artery for the pulse wave based on the plurality of pieces of transmural pressure.

7. The smartwatch according to any one of claims 1 to 6, wherein the amplitude of the pulse wave comprises at least one of the following: an amplitude of a main wave of the pulse wave, an amplitude of a tidal wave of the pulse wave, and an amplitude of a dicrotic wave of the pulse wave.

8. The smartwatch according to any one of claims 1 to 7, wherein there are a plurality of sensors, and the plurality of sensors form a sensor array.

9. A blood pressure measurement method, wherein the method is implemented by using a smartwatch, the smartwatch comprises a watch body, an airbag, and a sensor, the watch body is electrically connected to the sensor, the sensor is disposed on an inner wall of the airbag, and when the smartwatch is worn on a wrist of a user, the airbag and the sensor cover a radial artery of the wrist; and the method comprises:

   measuring a plurality of pulse waves of the radial artery through the sensor when the airbag is under different pressure strength; and determining blood pressure of the user based on the plurality of pulse waves through the watch body.

10. The method according to claim 9, wherein a distance between a top of a target area of the airbag and a top of the airbag is greater than a first threshold, a length of the target area is greater than a second threshold, a width of the target area is less than a product of a width of the airbag and a preset coefficient, and the target area is an area occupied by the sensor.

**11.** The method according to claim 9 or 10, wherein the method further comprises:

increasing pressure strength of the airbag through the watch body, so that the airbag squeezes the radial artery until the plurality of pulse waves of the radial artery from the sensor meet a preset condition, wherein the plurality of pulse waves are in one-to-one correspondence with a plurality of pieces of pressure strength of the airbag.

**12.** The method according to claim 11, wherein the adjusting pressure strength of the airbag through the watch body until the plurality of pulse waves of the radial artery from the sensor meet a preset condition comprises:

continuously increasing the pressure strength of the airbag through the watch body until an amplitude of a latest pulse wave in the plurality of pulse waves is less than a preset percentage of an amplitude of a target pulse wave, wherein the amplitude of the target pulse wave is the largest in amplitudes of the plurality of pulse waves; or intermittently increasing the pressure strength of the airbag through the watch body until an amplitude of a latest pulse wave in the plurality of pulse waves is less than an amplitude of a previous pulse wave; or continuously increasing the pressure strength of the airbag through the watch body until an amplitude of a latest pulse wave in the plurality of pulse waves is within a preset range.

**13.** The method according to any one of claims 9 to 12, wherein the determining blood pressure of the user based on the plurality of pulse waves through the watch body comprises:

determining a transfer function of the radial artery for a pulse wave and a transfer function of skin of the user for a pulse wave based on the amplitudes of the plurality of pulse waves through the watch body; and calculating the plurality of pulse waves based on the transfer functions through the watch body, to obtain the blood pressure of the user.

**14.** The method according to claim 13, wherein the determining a transfer function of the radial artery for a pulse wave based on the amplitudes of the plurality of pulse waves through the watch body comprises:

determining a plurality of pieces of transmural pressure of the radial artery based on the amplitudes of the plurality of pulse waves and a correspondence between an amplitude of a pulse wave and transmural pressure through the watch body; and determining the transfer function of the radial artery for the pulse wave based on the plurality of pieces of transmural pressure through the watch body.

**15.** The method according to any one of claims 9 to 14, wherein the amplitude of the pulse wave comprises at least one of the following: an amplitude of a main wave of the pulse wave, an amplitude of a tidal wave of the pulse wave, and an amplitude of a dicrotic wave of the pulse wave.

**16.** The method according to any one of claims 9 to 15, wherein there are a plurality of sensors, and the plurality of sensors form a sensor array.

**17.** A smartwatch, wherein the smartwatch comprises a memory and a processor, the memory stores code, the processor is configured to execute the code, and when the code is executed, the smartwatch performs the method according to any one of claims 9 to 16.

**18.** A computer storage medium, wherein the computer storage medium stores one or more instructions, and when the instructions are executed by one or more computers, the one or more computers are enabled to perform the method according to any one of claims 9 to 16.

**19.** A computer program product, wherein the computer program product stores instructions, and when the instructions are executed by a computer, the computer is enabled to perform the method according to any one of claims 9 to 16.

Watch body 100 of a smartwatch

| Memory | 105 |
| --- |
| Application 163 |

**Operating system 161**
- Application framework
- Library and runtime
- Kernel

107

Modem (Modem)

109

Radio frequency module

111

Wi-Fi module

101

Application processor (AP)

150

Positioning module

113

Bluetooth module

103

Microcontroller unit (MCU)

114

**Sensor**

| Light sensor | Motion sensor | ... |

**Input/Output (I/O) device**

151 — Display
153 — Touchscreen
155 — Audio circuit
116
117
115

FIG. 1

Watch body

Wrist of a user

Watchband

Radial bone

Ulnar bone

Radial artery

Ulnar artery

Airbag

Sensor

FIG. 2

Airbag

$x$

$h$

Target area

$a$

$b$

$w$

FIG. 3

Radial artery

One sensor

FIG. 4

Radial artery

One-dimensional array formed
by three sensors

FIG. 5

Radial artery

Two-dimensional array formed
by eight sensors

FIG. 6

A watch body of a smartwatch adjusts pressure strength of an airbag, so that the airbag squeezes a radial artery until a plurality of pulse waves of the radial artery from a sensor meet a preset condition, where the plurality of pulse waves are in one-to-one correspondence with a plurality of pieces of pressure strength of the airbag

701

The watch body determines blood pressure of a user based on the plurality of pulse waves

702

FIG. 7

Pressure strength of an airbag (mmHg)

Time (s)

FIG. 8

FIG. 9

FIG. 10

FIG. 11

FIG. 12

Main wave

Tidal wave

Dicrotic wave

FIG. 13

Radial
artery

Ulnar
artery

1  2  3

Wrist

FIG. 14a

Sensor 1    Sensor 2

Sensor 3

Radial artery

FIG. 14b

Processor

Inflation module — 1501

Determining module — 1502

FIG. 15

## INTERNATIONAL SEARCH REPORT

| International application No. |
|---|
| **PCT/CN2023/125632** |

| A. | CLASSIFICATION OF SUBJECT MATTER |
|---|---|

A61B5/022(2006.01)i;  A61B5/021(2006.01)i

According to International Patent Classification (IPC) or to both national classification and IPC

| B. | FIELDS SEARCHED |
|---|---|

Minimum documentation searched (classification system followed by classification symbols)

IPC:  A61B

Documentation searched other than minimum documentation to the extent that such documents are included in the fields searched

Electronic data base consulted during the international search (name of data base and, where practicable, search terms used)

DWPI, CNTXT, ENTXT: 华为, 手表, 智能, 穿戴, 腕表, 腕带, 手环, 血压, 测, 脉搏波, 桡动脉, 气囊, 气袋, 袖带, 传感, 感测, 加压, watch, smart, wear+, wristwatch, wristband, bracelet, ring, blood pressure, BP, measur+, pulse, wave, radial artery, bag, bladder, cuff, sens+, pressur+

| C. | DOCUMENTS CONSIDERED TO BE RELEVANT |
|---|---|

| Category* | Citation of document, with indication, where appropriate, of the relevant passages | Relevant to claim No. |
|---|---|---|
| X | CN 110099607 A (OMRON TATEISI ELECTRONICS CO., et al.) 06 August 2019 (2019-08-06) <br> description, paragraphs 0077-0133, and figures 1-14 | 1-4, 7-8, 17-19 |
| A | CN 106955091 A (SHANGHAI YILONG APPLICATION SOFTWARE DEVELOPMENT CO., LTD.) 18 July 2017 (2017-07-18) <br> entire document | 1-8, 17-19 |
| A | CN 113520357 A (HUAWEI TECHNOLOGIES CO., LTD.) 22 October 2021 (2021-10-22) <br> entire document | 1-8, 17-19 |
| A | CN 203000918 U (SHENYANG CITY WINLION MEDICAL ELECTRONIC CO., LTD.) 19 June 2013 (2013-06-19) <br> entire document | 1-8, 17-19 |
| A | US 2004158162 A1 (COLIN MEDICAL TECHNOLOGY CORPORATION) 12 August 2004 (2004-08-12) <br> entire document | 1-8, 17-19 |

☑ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **30 November 2023** | **19 December 2023** |

| Name and mailing address of the ISA/CN | Authorized officer |
|---|---|
| **China National Intellectual Property Administration (ISA/CN)** <br> **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

International application No.

**PCT/CN2023/125632**

**C.    DOCUMENTS CONSIDERED TO BE RELEVANT**

| Category* | Citation of document, with indication, where appropriate, of the relevant passages | Relevant to claim No. |
|---|---|---|
| A | US 2010286538 A1 (SAMSUNG ELECTRONIC CO., LTD.) 11 November 2010 (2010-11-11)<br>      entire document | 1-8, 17-19 |

**INTERNATIONAL SEARCH REPORT**

| International application No. |
| --- |
| **PCT/CN2023/125632** |

| Box No. II      Observations where certain claims were found unsearchable (Continuation of item 2 of first sheet) |
| --- |

This international search report has not been established in respect of certain claims under Article 17(2)(a) for the following reasons:

1. ☑ Claims Nos.: **9-16**
because they relate to subject matter not required to be searched by this Authority, namely:

The technical solutions of claims 9-16 relate to a blood pressure measurement method, which relates to a diagnostic method implemented on a human body, and falls within subject matter for which no search is required by the International Searching Authority as defined in PCT Rule 39.1(iv).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

## INTERNATIONAL SEARCH REPORT
### Information on patent family members

International application No.

**PCT/CN2023/125632**

| Patent document cited in search report | | | Publication date (day/month/year) | Patent family member(s) | | | Publication date (day/month/year) |
|---|---|---|---|---|---|---|---|
| CN | 110099607 | A | 06 August 2019 | US | 2019307336 | A1 | 10 October 2019 |
| | | | | JP | 2018102781 | A | 05 July 2018 |
| | | | | JP | 6761337 | B2 | 23 September 2020 |
| | | | | WO | 2018123243 | A1 | 05 July 2018 |
| | | | | DE | 112017006643 | T5 | 26 September 2019 |
| CN | 106955091 | A | 18 July 2017 | | None | | |
| CN | 113520357 | A | 22 October 2021 | WO | 2021208745 | A1 | 21 October 2021 |
| CN | 203000918 | U | 19 June 2013 | | None | | |
| US | 2004158162 | A1 | 12 August 2004 | JP | 2004223101 | A | 12 August 2004 |
| | | | | JP | 3795866 | B2 | 12 July 2006 |
| | | | | US | 7361148 | B2 | 22 April 2008 |
| US | 2010286538 | A1 | 11 November 2010 | KR | 20100120972 | A | 17 November 2010 |
| | | | | KR | 101577342 | B1 | 15 December 2015 |
| | | | | US | 8747327 | B2 | 10 June 2014 |

Form PCT/ISA/210 (patent family annex) (July 2022)

**REFERENCES CITED IN THE DESCRIPTION**

**Patent documents cited in the description**

- CN 202211312253